# EUROPEAN PATENT SPECIFICATION

(11) **EP 4 399 977 B1**
(45) Date of publication and mention of the grant of the patent: **31.12.2025**
(21) Application number: 23835560.6
(22) Date of filing: 05.07.2023
(51) Int. Cl.: A23K 20/163, A23K 50/10, A23K 50/30, A23K 50/60, A61K 31/7016, A61K 31/702, A61P 37/04

(54) **IGA PRODUCTION PROMOTER AND IGA PRODUCTION-PROMOTING FEED COMPOSITION**
IGA-PRODUKTIONSPROMOTOR UND IGA-PRODUKTIONSFÖRDERNDE FUTTERZUSAMMENSETZUNG
PROMOTEUR DE PRODUCTION D'IGA ET COMPOSITION D'ALIMENTATION FAVORISANT LA PRODUCTION D'IGA

(30) Priority: 08.07.2022 JP 2022110155
(43) Date of publication of application: 17.07.2024
(73) Proprietor: Nippon Beet Sugar Manufacturing Co., Ltd., Tokyo 1040031 (JP)
(72) Inventor: NAKAMURA, Mizuki, Obihiro-shi, Hokkaido 080-0831 (JP)
(74) Representative: Mewburn Ellis LLP
(86) International application number: PCT/JP2023/024912
(87) International publication number: WO 2024/010029

(56) References cited:
- JP-A- 2008 231 066
- JP-B2- 4 880 508
- JP-B2- 5 345 751

## Description

### Technical Field

The present invention relates to an IgA production promoting agent and a feed composition for promoting IgA production.

### Background Art

Health conditions of baby animals in suckling period are affected by various factors such as poor feeding or hygiene management, and pathogenic infections. In particular, as baby animals in suckling period have a high fatality rate due to diarrhea, pneumonia and the like, it is imperative to prevent diseases such as diarrhea in the management of baby animals in suckling period.

Mammals have an advanced immune system, but the immune function immediately after birth is immature and specific immunity defense mechanism (acquired immunity) such as antibody production ability is not functioning, whereby the defense ability against infectious diseases is poor. Under such a circumstance, mammal newborn animals ingest milk (colostrum) secreted from mother for about several days after delivery to receive nutrients such as proteins, lipids, sugars, and vitamins, and also immune components contained in the colostrum, such as immunoglobulins, thereby utilizing for health maintenance until own sufficient immune function is acquired.

The immunity of baby animals in suckling period includes passive immunity due to transferred maternal antibody and the like which a baby animal passively receives from a mother animal, and active immunity obtained by immune responses such as antibody production a baby animal itself induces. Immunoglobulins (antibodies) secreted in the colostrum of a mother animal typically reach epithelial cells of the intestinal tract of a newborn animal who ingested the colostrum, are absorbed by the epithelial cells, taken into the blood, and works as infection protective antibodies for a certain period of time. The transferred maternal antibody (passively-acquired antibody) gradually diminishes and disappears, whereas an antibody produced by a baby animal itself (actively-acquired antibody) increases. For example, calves gradually start producing antibodies some time after birth, and the blood antibody concentration is said to reach, at 3 months old, the level of an adult cattle. The period from 2 to 4 weeks old of calves is the bottom between the decreasing phase of passively-acquired antibodies and the ascending phase of actively-acquired antibodies, during which the calves are likely to have the declined immune defense ability and susceptible to pathogenic infections.

The transfer pathway of immunoglobulins from mother to child is known to vary depending on animal species. Primates including human have such a transfer through the colostrum after birth, but the transfer through the placenta in the womb accounts for a large proportion. On the other hand, in cattle, horse, pig, sheep, goat and the like, most of the immunoglobulins transfers to child through the colostrum, not through the placenta, thereby making the colostrum ingestion extremely important for the immune defense of newborn babies of these animals. The ability to absorb the immunoglobulins in the colostrum for a mammal newborn animal rapidly diminishes as time proceeds after birth. For this reason, it is considered desirable to allow such a newborn animal to ingest the colostrum from very early on after birth. The immunoglobulin absorption ability of a newborn calf is particularly high within 6 hours after birth, and when 24 hours to 48 hours have passed after birth, the immunoglobulins are poorly absorbed from the intestinal tract.

Five known animal-produced immunoglobulins include immunoglobulin G (IgG), immunoglobulin A (IgA), immunoglobulin E (IgE), immunoglobulin M (IgM), and immunoglobulin D (IgD). Of the immunoglobulins, IgG accounts for a significant proportion, but it is IgA that plays the major role in the mucosal immunity such as intestinal immunity that has been drawing attention in recent years. IgA forms a multimer including a dimer, is secreted in the form of secretory IgA, and captures pathogens and foreign matters on the mucosa to prevent them from invading the body. Secretory IgA is resistant to degradation by a digestive enzyme and protects the intestinal mucosa in the intestinal tract, thereby preventing pathogens from invading through the intestinal tract wall. For this reason, secretory IgA plays an important role in preventing diarrhea of a baby animal in suckling period (Non Patent Literature 1). However, most of the immunoglobulins in the colostrum of cow and the like is IgG, and the IgA content in the colostrum is small. It is thus considered effective to promote the IgA production at an early stage for preventing infectious diarrhea in baby animals in suckling period.

As an IgA production promoting substance, malted rice (rice koji) (Patent Literature 1), lactic acid bacteria (Patent Literature 2), galactooligosaccharides (Patent Literature 3), fructooligosaccharides (Non Patent Literature 2), whey protein (Non Patent Literatures 1 and 3), β-carotene (Non Patent Literature 4) and the like have been reported. However, there are no reports of IgA production promoting substances capable of providing stimulation of intestinal immunity from an early stage in suckling period.

Patent Literature 4 discloses that difructose anhydride III and difructose anhydride IV promote the absorption of immunoglobulins (in particular, IgG) from the colostrum in mammal newborn animals. However, the immunoglobulin absorption ability of newborn animals rapidly diminishes as time proceeds after birth, and simultaneously the immunoglobulin concentration in breast milk also significantly diminishes as time proceeds after delivery. For this reason, the period during which the immunoglobulin absorption promotion is effective in newborn animals is short. For more effective reinforcement in the infection protection of newborn animals, early activation of own immune function in newborn animals is desirable.

### Citation List

### Patent Literature

Patent Literature 1: JP Patent Publication No. 2018-118967 A
Patent Literature 2: JP Patent Publication No. 2007-308419 A
Patent Literature 3: JP Patent Publication No. 2006-298783 A
Patent Literature 4: JP Patent Publication No. 2008-231066 A

### Non Patent Literature

Non Patent Literature 1: Yasumatsuya et al., Animal Science Journal 84 (3), 389-393, 2013
Non Patent Literature 2: Nakamura et al., Digestive Organ and Mucosal Immunology, No. 40, 38-40, 2003
Non Patent Literature 3: Yasumatsuya K, et al., Livest. Sci., (2012) 143:210-213.
Non Patent Literature 4: Otomaru et al., Journal of the Japan Veterinary Medical Association, 72, 344-347, 2019

### Summary of Invention

### Technical Problem

The present invention has a problem to provide an IgA production promoting substance effective even in suckling period.

### Solution to Problem

The present inventors conducted extensive studies to solve the above problem and found that difructose anhydride III is capable of promoting IgA production from an early stage after birth, whereby the present invention has been accomplished.

Specifically, the present invention is as defined in the appended claims.

### Advantageous Effects of Invention

According to the present invention, the IgA production in animals can be effectively promoted.

### Description of Embodiments

Hereinafter, the present invention will be described in detail. Reference to methods of treatment should be understood to refer to products for use in such methods. Embodiments outside the scope of the claims are for illustrative purposes only.

The present invention relates to a technique for immune stimulation based on the activation of intestinal immune system (stimulation of intestinal immunity). The present invention relates to, in particular, a technique for promotion of immunoglobulin A (IgA) production. The present invention also relates to a technique for promotion of IgA production in animals in suckling period. More specifically, the present invention relates to use of difructose anhydride III for stimulation of intestinal immunity in animals, in particular, for IgA production promotion.

Difructose anhydride (DFA) is a collective term of reducing disaccharide compounds in which the reducing ends of two fructoses mutually bind to hydroxyl groups other than the reducing end of the other fructose. Difructose anhydride III (DFAIII), a type of the difructose anhydride, is a disaccharide in which two fructose molecules are bonded together at the positions 1, 2 and 2, 3', and is also written as di-D-fructofuranose 1, 2:2,3' dianhydride. DFAIII is known to be contained in caramel, honey and the like, and is industrially produced by enzymatically treating inulin derived from chicory or the like with fructotransferase (EC 2.4.1.93) derived from *Arthrobacter* sp. H65-7 strain. However, DFAIII to be used in the present invention is not limited to those obtained by a specific production method.

The present invention provides an immunoglobulin A (IgA) production promoting agent comprising DFAIII. The present invention also provides DFAIII or an agent or a composition comprising DFAIII, for promoting IgA production. The present invention also provides a feed composition , in particular, a feed composition for promoting IgA production, comprising DFAIII. The IgA production promoting agent and the feed composition according to the present invention can comprise DFAIII as an active ingredient. In the present invention, the "comprising DFAIII as an active ingredient" means that DFAIII is contained in an amount (effective amount) and in composition that is capable of providing the intended effect (for example, the effect to promote IgA production). The IgA production promoting agent and the feed composition according to the present invention can contain an effective amount of DFAIII.

In the present invention, the animal as a subject for the IgA production promotion is cattle.

In one embodiment, the IgA production promoting agent and the feed composition according to the present invention may be for an animal in suckling period. In the present invention, the "suckling period" means the period from immediately after birth to weaning, and specifically refers to the period during which a mammal is fed milk (for example, breast milk and/or milk substitute) once a day or more as a nutrient source. For example, the weaning age for cattle varies depending on the breed, method for rearing, growth conditions of individuals and the like, but dairy cattle commonly wean at about 6 weeks to 2 months old (or about 3 months old in some cases). However, the IgA production promoting agent and the feed composition according to the present invention are not limited to those for animals in suckling period, and may be used in non-suckling period (after weaning).

Mammal newborn animals have immature digestive organs and are incapable of digesting and absorbing solids well. In the livestock industry, it is common to feed the colostrum for about several days to about 10 days after birth, and then feed a milk substitute instead of breast milk to a baby animal such as a calf, so as to achieve early weaning of the animal for the purpose of productivity improvement. The "colostrum" refers to the milk secreted from mother for a period from just after delivery to about several days (about 10 days at longest) after delivery. The colostrum contains various components, such as immunoglobulins and physiologically active substances, in addition to nutrients. In particular, the colostrum immediately after delivery contains a high concentration of immunoglobulins. The "milk substitute" refers to a feed that is fed in the form of a liquid as a substitute for breast milk, and contains nutrients, additives and the like required for the growth and the like of baby animals. Generally, a milk substitute available in the form of a powder (milk substitute in powder) is dissolved in hot water or water and then fed as the milk substitute, but the milk substitute is not limited to such a form.

In the present invention, the "breast milk" means the milk secreted from an animal individual which is in breastfeeding period after delivery. The "breast milk" is not limited to the milk from a mother individual which has a genetic parent- child relationship with a child to be fed, unless the context clearly means otherwise. Similarly, the "colostrum" is not limited to the colostrum from a mother individual which has a genetic parent-child relationship with a child to be fed, unless the context clearly means otherwise.

The IgA production promoting agent according to the present invention may contain only DFAIII, or may be a mixture of DFAIII dissolved in or mixed (or otherwise) with a medium such as water, or may be a composition comprising DFAIII, but are not limited thereto. In one embodiment, the IgA production promoting agent according to the present invention may be a pharmaceutical composition. In one embodiment, the IgA production promoting agent according to the present invention may be a nutritional aid (supplement and the like) or a feed additive.

The IgA production promoting agent according to the present invention may contain, in addition to DFAIII, any pharmaceutically acceptable additives (excipients), such as an inert carrier (solid carrier and liquid carrier), a filler, a diluent, a stabilizer, a surfactant, a binder, a disintegrant, a lubricant, a solubilizing agent, a suspending agent, a coating agent, a coloring agent, an aromatic substance, a flavoring agent, an emulsifier, a preservative, a buffer, a pH adjuster, and an antioxidant. The IgA production promoting agent according to the present invention may further contain other substances such as different immunostimulants and/or pharmacological substances.

The IgA production promoting agent according to the present invention may be prepared into any dosage form, including solid preparations such as a tablet, a granule, a powder, a pill, a capsule, and a pellet, liquid preparations such as a solution, a suspension, and a syrup, a paste, a gel, and an aerosol. The IgA production promoting agent may also be prepared into a preparation to be reconstituted before use, such as a dry product which is dissolved in or mixed with a liquid medium immediately before use to reconstitute into a liquid preparation.

The IgA production promoting agent according to the present invention may be used for any administration/feeding route, for example, oral route, or any parenteral route such as enteral or intravenous route, and preferably for oral or enteral route. Examples of enteral administration/feeding route include tube feeding such as nasal feeding and gastric fistula feeding, and rectal administration (suppository), but are not limited thereto.

The feed composition according to the present invention contains DFAIII, in addition to any feed raw materials such as grains, oil meals, brans, and/or coarse feeds, or any feed products. The feed composition according to the present invention may further contain any feed additives acceptable in feed production, such as an inert carrier (solid carrier and liquid carrier), a filler, a diluent, a stabilizer, a surfactant, a binder, a disintegrant, a lubricant, a solubilizing agent, a suspending agent, a coating agent, a coloring agent, an aromatic substance, a flavoring agent, an emulsifier, a preservative, a buffer, a pH adjuster, an antioxidant, amino acids, vitamins, minerals, an antibacterial agent, an antibiotic, a seasoning, an enzyme, an organic acid, and a probiotic. The feed composition according to the present invention may further contain other substances such as different immunostimulants and pharmacological substances. In one embodiment, the feed composition according to the present invention may contain an existing or commercially available feed such as a mixed feed for animals in suckling period (also called a starter) and a milk powder preparation (for example, a colostrum powder preparation or breast milk powder preparation). In one embodiment, the feed composition according to the present invention may contain the colostrum or breast milk, and may contain, for example, a colostrum powder or a breast milk powder. In another embodiment, the feed composition according to the present invention does not contain breast milk, and particularly does not contain the colostrum.

The feed composition according to the present invention may be obtained simply by mixing raw materials, or may be molded or prepared in any shape such as solids (powder form/mashed form, granular form, pellet form, crumble form, flake form, bulky form and the like), semi-solids, and liquids. In one embodiment, the feed composition according to the present invention may be molded or prepared as a feed product such as a starter or a coarse feed by adding DFAIII to feed raw materials such as a starter, a coarse feed or the like.

The feed composition according to the present invention contains DFAIII preferably in an amount of 0.1 wt% or more, more preferably in an amount of 0.1 to 10 wt%, and further preferably in an amount of 0.5 to 5 wt%, and for example, in an amount of 1 to 4 wt%, relative to the total weight of the feed composition (wet weight at the time of mixing raw materials), but is not limited thereto. Regarding the DFAIII content in the feed composition, wt% may be used interchangeably with w/w% [weight/weight %].

The IgA production promoting agent and the feed composition according to the present invention are capable of providing stimulation of intestinal immunity in animals due to the action of DFAIII, and promoting the IgA production. The IgA production promoting agent and the feed composition according to the present invention can be used for the stimulation of intestinal immunity.

In a preferred embodiment, the IgA production promoting agent and the feed composition according to the present invention are capable of providing the stimulation of intestinal immunity and promoting the IgA production due to the action of DFAIII even in suckling period during which the immune function is considered immature. An animal, which is particularly in early suckling period within suckling period, which is a period that is the bottom between the decreasing phase of passively-acquired antibodies derived from the colostrum and the increasing phase of actively-acquired antibodies (immunity level declining period) or in a period before the period, is particularly preferred as a subject for administration or feeding of the IgA production promoting agent or the feed composition according to the present invention. For example, in the case of cattle, the period that is the bottom between the decreasing phase of passively-acquired antibodies derived from the colostrum and the increasing phase of actively-acquired antibodies, is typically 2 to 4 weeks old (14 to 28 days old). In animals, for example, ungulates such as cattle, the period that is the bottom between the decreasing phase of passively-acquired antibodies derived from the colostrum and the increasing phase of actively-acquired antibodies, or the period before the period, which is a particularly preferable period for administering or feeding the IgA production promoting agent or the feed composition according to the present invention, may be, for example, between immediately after birth and 28 days old, or 5 days old to 28 days old, but is not limited thereto. The period that is the bottom between the decreasing phase of passively-acquired antibodies derived from the colostrum and the increasing phase of actively-acquired antibodies, or the period before the period, is particularly preferable as an administration or feeding timing, but the IgA production promoting agent or the feed composition according to the present invention may be administered or fed, in addition to these periods or instead of these periods, during other periods (later suckling period, non-suckling period and the like). In the present invention, the number of age written as "X" days old" or the like means the number of age calculated with taking the day of birth as day 0, and the following day after birth as 1 day old.

The present invention provides a method for promoting IgA production in the above-mentioned animals as a subject therefor, comprising administering the IgA production promoting agent according to the present invention to the subject. The present invention also provides a method for promoting IgA production in the above-mentioned non-human animals as a subject therefor, comprising feeding the feed composition according to the present invention to the animal. In the present invention, the DFAIII administration or feeding can provide stimulation of intestinal immunity and promote the IgA production in animals..

In the present invention, the "administration" of the IgA production promoting agent may be, as described above, an administration by any administration route, including oral administration, or any parenteral administrations such as enteral administration or intravenous administration, and preferably by oral administration or enteral administration. The IgA production promoting agent may be, for example, administered by direct introduction to the mouth, or into a tube or the like for tube feeding. Alternatively, the IgA production promoting agent may be added to drinking water, the colostrum, breast milk, or a solution of a milk powder preparation dissolved in water, or added to a feed, and administered to a non-human animal by ingestion thereof. The IgA production promoting agent formulated into a pharmaceutical product may be administered to a subject. The IgA production promoting agent may be administered after reconstitution into a liquid preparation or the like before use.

In the present invention, the "feeding" of the feed composition means providing the feed composition as a feed to the animal to allow ingest it. The feed composition may be fed by free feeding, fed at a set feeding amount, feeding time the like, or force-fed. The feed composition according to the present invention may be fed after completion of the colostrum feeding period. The feeding of the feed composition according to the present invention may start at, for example, 0 days old, 1 day old, 2 days old, 3 days old, 4 days old, 5 days old, 6 days old, 7 days old, 8 days old, or even after that.

A calf in suckling period is commonly fed the colostrum over a period of about several days after birth, and then fed a milk substitute instead of breast milk. Typically, the milk substitute is fed about twice a day to a calf at about 1 week old to about 2 months old. However, feeding a milk substitute alone fails to sufficiently develop the ruminant stomach of a calf. For this reason, ingestion of a solid feed such as a mixed feed for calf in suckling period, i.e., a starter, by a calf in addition to the milk substitute, is used for feeding management to promote the development of the ruminant stomach. Additionally, when a calf in suckling period is reared with breast milk, it is commonly practiced to feed a solid feed such as a starter and/or a coarse feed, in combination with breast milk, to promote growth. Similarly, in the present invention, a non-human animal in suckling period as a subject for the administration of the IgA production promoting agent or the feeding of the feed composition according to the present invention may be fed a solid feed such as a starter, or other feeds such as coarse feeds including hay, fermented fibrous materials (silage) and the like, in addition to the feeding of a milk substitute and/or breast milk. The administration of the IgA production promoting agent according to the present invention to a non-human animal in suckling period may be performed by adding the IgA production promoting agent according to the present invention to a feed such as a milk substitute, a starter, and/or a coarse feed to be given to the non-human animal in suckling period, and giving it thereto. The feeding of the feed composition according to the present invention to a non-human animal in suckling period may be performed by mixing the feed composition according to the present invention with a feed such as a milk substitute, a starter, and/or a coarse feed, and giving it thereto.

The IgA production promoting agent or the feed composition according to the present invention is administered or fed continuously (for example, daily or every other day; and/or once a day or more, or free feeding), and more preferably administered or fed daily. In a preferred embodiment, the administration of the IgA production promoting agent or the feeding of the feed composition according to the present invention can be performed or started at an early stage after birth. In the present invention, the administration or feeding of DFAIII at an early stage after birth can achieve the stimulation of intestinal immunity and the IgA production promotion at an early stage. The administration of the IgA production promoting agent or the feeding of the feed composition according to the present invention is preferably performed or started in suckling period for mammals, and more preferably performed or started particularly in early suckling period, which is the bottom between the decreasing phase of passively-acquired antibodies derived from the colostrum and the increasing phase of actively-acquired antibodies, or before the period. In the case of cattle, the period of the bottom between the decreasing phase of passively-acquired antibodies derived from the colostrum and the increasing phase of actively-acquired antibodies is about 2 to 4 weeks old. The administration of the IgA production promoting agent or the feeding of the feed composition according to the present invention is preferably performed at least in the period of the bottom between the decreasing phase of passively-acquired antibodies derived from the colostrum and the increasing phase of actively-acquired antibodies, or before the period.

The administration of the IgA production promoting agent or the feeding of the feed composition according to the present invention is performed to cattle, at leastbetween 7 days old and 28 days old; and preferably at least between immediately after birth (0 or 1 day old; the same applied hereafter) and 28 days old (4 weeks old), or between 5 days old and 28 days old. In one embodiment, the administration of the IgA production promoting agent or the feeding of the feed composition according to the present invention may be performed to cattle continuously (for example, daily or every other day), between immediately after birth and 28 days old or between 5 days old and 28 days old. The administration of the IgA production promoting agent or the feeding of the feed composition according to the present invention is preferably started in the period which is the bottom between the decreasing phase of passively-acquired antibodies derived from the colostrum and the increasing phase of actively-acquired antibodies, or before the period.

In one embodiment, the administration of the IgA production promoting agent or the feeding of the feed composition according to the present invention to cattle, and particularly in suckling period, may be started between immediately after birth and 4 days old (for example, immediately after birth), and may be started, for example, immediately after birth, at 5 days old, or 7 days old, between immediately after birth and 7 days old, or between 5 days old and 7 days old.

In one embodiment, the administration of the IgA production promoting agent or the feeding of the feed composition according to the present invention to cattle is performed from the start of the administration or feeding and preferably to any completion time (DFAIII administration or feeding completion time) set after the period that is the bottom between the decreasing phase of passively-acquired antibodies derived from the colostrum and the increasing phase of actively-acquired antibodies, at least up to 28 days old (4 weeks old/1 month old), at least up to 8 weeks old (56 days old/2 months old), or at least up to 12 weeks old (84 days old/3 months old).

In one embodiment, the administration of the IgA production promoting agent or the feeding of the feed composition according to the present invention may be performed continuously until the level of actively-acquired antibodies reaches the adult level.

In one embodiment, the administration of the IgA production promoting agent or the feeding of the feed composition according to the present invention to cattle may be performed in the period between immediately after birth to 4 days old (for example, immediately after birth), and then further performed at 5 days old or 7 days old and thereafter.

In one embodiment, the administration of the IgA production promoting agent or the feeding of the feed composition according to the present invention to cattle, and particularly in suckling period may be started between immediately after birth to 4 days old (for example, immediately after birth), and then performed continuously at least up to 28 days old. In the present invention, both of the administration of the IgA production promoting agent according to the present invention and the feeding of the feed composition according to the present invention may be carried out.

In one embodiment, for example, cattle may be fed the colostrum to which the IgA production promoting agent is added, during the colostrum feeding period of about several to 10 days from immediately after birth (for example, of up to 6 days old after birth), and then fed the feed composition according to the present invention after completion of the colostrum feeding. Regarding the present invention, the "completion" of colostrum feeding means that after the colostrum is fed for a certain period of time after birth to a mammal newborn animal, further colostrum feeding to the newborn animal is terminated. The performing "continuously" the administration of the IgA production promoting agent or the feeding of the feed composition according to the present invention means the giving of DFAIII by the administration or feeding for a certain period of time, and regularly, intermittently, or persistently. For example, the case in which the IgA production promoting agent according to the present invention is administered (for example, administered multiple times between immediately after birth to 4 days old), and then the feed composition according to the present invention is fed (for example, fed daily from 5 to 7 days old and thereafter) can also belong to the "performing "continuously" the administration of the IgA production promoting agent or the feeding of the feed composition according to the present invention.

Immune system includes the "mucosal immunity" that prevents pathogens such as viruses and bacteria and foreign matters from invading the body, and the "systemic immunity" that functions to eliminate pathogens and foreign matters when they invade the body. In the mucosal immunity, a physical barrier is formed so that it physically prevents the invasions of pathogens and foreign matters by mucus and strong intercellular tight junction, and a chemical barrier is also formed in which the secretory IgA secreted on the mucosal surface binds to pathogens and foreign matters to block the invasion of the body and then promote excretion thereof from the body and bactericidal peptides, lysozyme and the like are secreted to eliminate pathogens. In particular, secretory IgA plays an important role of infection protection in the mucosal immunity. The mucosal immunity functions on the mucosal surface of the entire body, and the mucosal immunity in the intestinal tract, in other words, the intestinal immunity, plays the central role of the mucosal immunity. Antigen-stimulated B cells in the mucosa-related lymphoid tissues present in the intestinal tract differentiate into IgA-producing cells that produce the secretory IgA antibody, and the IgA-producing cells travel for horming to distal other mucosal tissues and produce secretory IgA antibody therein. As a result, due to the action of DFAIII, the same antigen-specific immune response is induced not only in the antigen-stimulated mucosal tissues but also in distal mucosal tissues. In the present invention, the intestinal immunity can be effectively activated and the IgA production can be promoted in the mucosal tissues including the intestinal mucosa.

The IgA concentration in feces has been commonly used as an indicator for intestinal immune function. Thus, the stimulation of intestinal immunity and the IgA production promotion in the present invention can be evaluated using the IgA concentration in feces as an indicator. The IgA concentration in feces can be expressed as an amount (milligram) of IgA per wet weight (g) of feces. A test sample (in the present invention, DFAIII, or the IgA production promoting agent or the feed composition comprising DFAIII) is administered or fed to an animal, an IgA concentration in feces of the animal is determined, and if the IgA concentration is increased in comparison with an IgA concentration in feces of a control animal to which the test sample is not administered or fed, the result indicates that the test sample promotes the IgA production. For example, if in the IgA concentration in feces of an animal to which a test sample is administered or fed is increased in comparison with an IgA concentration in feces of a control animal to which the test sample is not administered or fed, with a statistically significant difference at P<0.05, or if the IgA concentration in feces of an animal to which a test sample is administered or fed is increased by 50% or more in comparison with an IgA concentration in feces of a control animal to which the test sample is not administered or fed, the test sample can be determined to "have increased" the IgA concentration in feces in the animal to which the test sample is administered or fed in comparison with the IgA concentration in feces of the control animal to which the test sample is not administered or fed. Also, if the IgA production promotion is shown by using the IgA concentration in feces being as an indicator, the intestinal immunity can be determined to have been stimulated.

Further, the present invention also provides use of DFAIII in the above-mentioned animals, preferably animals in suckling period, for the stimulation of intestinal immunity or the IgA production promotion (for example, IgA production promotion in the intestinal mucosa). The present invention also provides use of DFAIII in the above-mentioned animals, preferably animals in suckling period, for preventing infections such as infectious diarrhea or reinforcing the infection protection ability by the IgA production promotion. The present invention also provides use of DFAIII in the production of a medicament or a feed for the stimulation of intestinal immunity or the IgA production promotion (for example, IgA production promotion in the intestinal mucosa).

### Example

Hereinafter, the present invention will be further specifically described in reference to Examples. However, the technical scope of the present invention is not limited to these Examples.

### [Example 1]

Twelve head of Holstein calves in suckling period as test subjects were divided into a control group (7 head) and a DFAIII feeding group (5 head). Colostrum was fed to the calves up to 6 days old after birth. In the DFAIII feeding group, 6 g each of difructose anhydride III (DFAIII) (manufactured by Nippon Beet Sugar Manufacturing Co., Ltd.; product name: Koushi no Mikata) was mixed with the colostrum and fed to the calves in the first and second colostrum feeding after birth (at 0 to 1 day old). From 7 days old, the 7 head of calves in suckling period in the control group were fed with a starter, which is a mixed feed for calf in suckling period (manufactured by Nippon Beet Sugar Manufacturing Co., Ltd.; product name: Start; component contents: protein 22%, total digestible nutrients 74%), and the 5 head of calves in suckling period in the DFAIII feeding group were fed with a feed (DFAIII-containing feed) in which DFAIII (manufactured by Nippon Beet Sugar Manufacturing Co., Ltd.) was added in an amount of 2.0 wt% to and mixed with the starter, by free feeding together with hay and water. The amount 2.0 wt% of DFAIII in the DFAIII-containing feed is calculated as the weight proportion (w/w%) of DFAIII relative to the total weight of the starter and DFAIII. From seven days old, 2 L of 15% milk substitute solution (without DFAIII) was further fed to the calves in suckling period twice a day.

Fresh feces were collected from the calves of each test group at 7 days old, 14 days old, 21 days old, and 28 days old. The IgA concentrations in feces were measured by the ELISA method using an IgA ELISA Quantitation Kit (manufactured by BETHYL Laboratories, Inc.). The IgA concentration in feces was expressed in a milligram amount of IgA per wet mass of the feces. The results are shown in Table 1.

**Table 1**

| | IgA Concentration (mg/g feces) | | | |
|---|---|---|---|---|
| | 7 days old | 14 days old | 21 days old | 28 days old |
| Control Group | 0.11 ± 0.07 | 0.43 ± 0.24 | 0.64 ± 0.65 | 0.24 ± 0.33 |
| DFAIII Feeding Group | 0.65 ± 0.32** | 1.19 ± 0.45** | 2.60 ± 1.01* | 1.01 ± 1.34 |

| | | | | |
|---|---|---|---|---|
| Data represents mean ± standard deviation. ** and * indicate that there is a significant difference between the control group and the feeding group, with significance levels p<0.01 and p<0.05, respectively. | | | | |

As shown in Table 1, the DFAIII feeding group had statistically significantly higher IgA concentrations in feces in comparison with the control group, thereby showing the promoted IgA production in the intestinal tract, that is, enhanced intestinal immunity.

In both of the test groups, however, the IgA concentrations in feces decreased at 28 days old in comparison with 21 days old. The factor therefor is considered, for example, the relative concentration decrease, due to the gradual decrease of the IgA from the colostrum at about 1 month old and the increased feces amount as the feed intake increases.

The above test results show that DFAIII is able to promote the IgA production even in animals in suckling period having immature intestinal immune system, thereby stimulating the mucosal immunity including the intestinal immunity.

### Industrial Applicability

The present invention may be used for providing an immunostimulating agent or a feed useful for providing intestinal immunity stimulation and the IgA production promotion. The present invention is particularly useful for, for example, activating the immature intestinal immune system of animals in suckling period such as baby animals in suckling period, and further for activating the systemic mucosal immune system. The present invention may be utilized for the purpose of preventing infectious diarrhea and pneumonia, to which animals in suckling period are susceptible, and enhancing the infection protection ability, by promoting the IgA production in the mucosa, thereby enhancing the immunity at an early stage in the immunity level-declining period of animals in suckling period so as to curtail the period. The present invention prevents the loss of young baby animals and promotes healthy growth thereof, and is thus beneficial for the livestock industry in the light of expectedly reducing the potential loss in the livestock farming.

## Claims

1. Difructose anhydride III for use as an active ingredient in a method of promoting immunoglobulin A (IgA) production in cattle, wherein the method comprises administering an IgA production promoting agent comprising difructose anhydride III as an active ingredient to the cattle continuously between 7 days old and 28 days old.

2. A feed composition for use in a method of promoting IgA production in cattle, comprising difructose anhydride III as an active ingredient, wherein the method comprises feeding the feed composition to the cattle continuously between 7 days old and 28 days old.

3. The feed composition for use according to claim 2, wherein the feed composition is solid.

4. The feed composition for use according to claim 2 or claim 3, wherein the feed composition further comprises a starter or a coarse feed.

5. Difructose anhydride III for use according to claim 1 or the feed composition for use according to any one of claims 2-4, wherein said use is in a method of promoting IgA production in the period of 2 to 4 weeks old of the cattle.

6. Difructose anhydride III for use according to claim 1 or claim 5, or the feed composition for use according to any one of claims 2-5, wherein said use is for stimulation of intestinal immunity to promote IgA production.

7. The feed composition for use according to any one of claims 2-6, wherein the feed composition comprises difructose anhydride III in an amount of 0.1 to 10 wt%.

8. Difructose anhydride III for use according to any one of claims 1 and 5-6 or the feed composition for use according to any one of claims 2-7, wherein said use is for preventing infection.

9. Difructose anhydride III for use according to claim 8 or the feed composition for use according to claim 8, wherein the infection is infectious diarrhea or pneumonia.

10. Difructose anhydride III for use according to claim 9 or the feed composition for use according to claim 9, wherein the infection is infectious diarrhea.

11. Difructose anhydride III for use according to any one of claims 1, 5-6 and 8-10 wherein administering an IgA production promoting agent comprising difructose anhydride III as an active ingredient to the cattle continuously between 7 days old and 28 days old comprises administering the IgA production promoting agent to the cattle daily, every other day, once a day or more or by free feeding.

12. The feed composition for use according to any one of claims 2-10, wherein feeding the feed composition to the cattle continuously between 7 days old and 28 days old comprises feeding the feed composition to the cattle daily, every other day, once a day or more or by free feeding.

## Patentansprüche

1. Difructoseanhydrid III zur Verwendung als Wirkstoff in einem Verfahren zum Fördern der Immunglobulin-A- (IgA-) Produktion bei Rindern, wobei das Verfahren das kontinuierliche Verabreichen eines die IgA-Produktion fördernden Mittels, das Difructoseanhydrid III als Wirkstoff umfasst, an die Rinder in einem Alter von zwischen 7 Tagen und 28 Tagen umfasst.

2. Futterzusammensetzung zur Verwendung in einem Verfahren zum Fördern der IgA-Produktion bei Rindern, die Difructoseanhydrid III als Wirkstoff umfasst, wobei das Verfahren das kontinuierliche Verfüttern der Futterzusammensetzung an die Rinder im Alter von zwischen 7 Tagen und 28 Tagen umfasst.

3. Futterzusammensetzung zur Verwendung nach Anspruch 2, wobei die Futterzusammensetzung fest ist.

4. Futterzusammensetzung zur Verwendung nach Anspruch 2 oder Anspruch 3, wobei die Futterzusammensetzung weiters ein Starterfutter oder ein grobes Futter umfasst.

5. Difructoseanhydrid III zur Verwendung nach Anspruch 1 oder Futterzusammensetzung zur Verwendung nach einem der Ansprüche 2 bis 4, wobei die Verwendung in einem Verfahren zum Fördern der IgA-Produktion im Zeitraum eines Alters der Rinder von 2 bis 4 Wochen erfolgt.

6. Difructoseanhydrid III zur Verwendung nach Anspruch 1 oder Anspruch 5 oder Futterzusammensetzung zur Verwendung nach einem der Ansprüche 2 bis 5, wobei die Verwendung zur Stimulation der Darmimmunität zum Fördern der IgA-Produktion dient.

7. Futterzusammensetzung zur Verwendung nach einem der Ansprüche 2 bis 6, wobei die Futterzusammensetzung Difructoseanhydrid III in einer Menge von 0,1 bis 10 Gew.-% umfasst.

8. Difructoseanhydrid **III** zur Verwendung nach einem der Ansprüche 1 und 5 bis 6 oder Futterzusammensetzung zur Verwendung nach einem der Ansprüche 2 bis 7, wobei die Verwendung zur Prävention einer Infektion dient.

9. Difructoseanhydrid III zur Verwendung nach Anspruch 8 oder Futterzusammensetzung zur Verwendung nach Anspruch 8, wobei die Infektion infektiöse Diarrhoe oder Pneumonie ist.

10. Difructoseanhydrid III zur Verwendung nach Anspruch 9 oder Futterzusammensetzung zur Verwendung nach Anspruch 9, wobei die Infektion infektiöse Diarrhoe ist.

11. Difructoseanhydrid III zur Verwendung nach einem der Ansprüche 1, 5 bis 6 und 8 bis 10, wobei das kontinuierliche Verabreichen eines die IgA-Produktion fördernden Mittels, das Difructoseanhydrid III als Wirkstoff umfasst, an die Rinder im Alter von zwischen 7 Tagen und 28 Tagen das Verabreichen des die IgA-Produktion fördernden Mittels täglich, jeden zweiten Tag, einmal täglich oder öfter oder durch freies Verfüttern an die Rinder umfasst.

12. Futterzusammensetzung zur Verwendung nach einem der Ansprüche 2 bis 10, wobei das kontinuierliche Verfüttern der Futterzusammensetzung an die Rinder im Alter zwischen 7 Tagen und 28 Tagen das Verfüttern der Futterzusammensetzung täglich, jeden zweiten Tag, einmal täglich oder öfter oder durch freies Verfüttern an die Rinder umfasst.

## Revendications

1. Anhydride de difructose III pour utilisation en tant que principe actif dans un procédé de promotion de la production d'immunoglobuline A (IgA) chez le bétail, dans lequel le procédé comprend l'administration d'un agent de promotion de production d'IgA comprenant de l'anhydride de difructose III en tant que principe actif au bétail en continu entre 7 jours et 28 jours.

2. Composition d'alimentation pour utilisation dans un procédé de promotion de la production d'IgA chez le bétail, comprenant de l'anhydride de difructose III en tant que principe actif, dans laquelle le procédé comprend l'alimentation du bétail avec la composition d'alimentation en continu entre 7 jours et 28 jours.

3. Composition d'alimentation pour utilisation selon la revendication 2, dans laquelle la composition d'alimentation est solide.

4. Composition d'alimentation pour utilisation selon la revendication 2 ou la revendication 3, dans laquelle la composition d'alimentation comprend en outre un aliment de démarrage ou du fourrage grossier.

5. Anhydride de difructose III pour utilisation selon la revendication 1 ou composition d'alimentation pour utilisation selon l'une quelconque des revendications 2 à 4, ladite utilisation étant dans un procédé de promotion de la production d'IgA dans la période de 2 à 4 semaines chez le bétail.

6. Anhydride de difructose III pour utilisation selon la revendication 1 ou la revendication 5, ou composition d'alimentation pour utilisation selon l'une quelconque des revendications 2 à 5, ladite utilisation étant pour la stimulation de l'immunité intestinale afin de favoriser la production d'IgA.

7. Composition d'alimentation pour utilisation selon l'une quelconque des revendications 2 à 6, dans laquelle la composition d'alimentation comprend de l'anhydride de difructose III en une quantité de 0,1 à 10 % en poids.

8. Anhydride de difructose III pour utilisation selon l'une quelconque des revendications 1 et 5 à 6 ou composition d'alimentation pour utilisation selon l'une quelconque des revendications 2 à 7, ladite utilisation étant pour prévenir une infection.

9. Anhydride de difructose III pour utilisation selon la revendication 8 ou composition d'alimentation pour utilisation selon la revendication 8, l'infection étant une diarrhée infectieuse ou une pneumonie.

10. Anhydride de difructose III pour utilisation selon la revendication 9 ou composition d'alimentation pour utilisation selon la revendication 9, l'infection étant une diarrhée infectieuse.

11. Anhydride de difructose III pour utilisation selon l'une quelconque des revendications 1, 5-6 et 8-10, dans lequel l'administration d'un agent favorisant la production d'IgA comprenant de l'anhydride de difructose III en tant que principe actif au bétail en continu entre 7 jours et 28 jours comprend l'administration de l'agent favorisant la production d'IgA au bétail tous les jours, tous les deux jours, une fois par jour ou plus ou par alimentation libre.

12. Composition d'alimentation pour utilisation selon l'une quelconque des revendications 2 à 10, dans laquelle l'alimentation de la composition d'alimentation au bétail en continu entre 7 jours et 28 jours comprend l'alimentation de la composition d'alimentation au bétail tous les jours, tous les deux jours, une fois par jour ou plus ou par alimentation libre.
